(19) Europäisches Patentamt
European Patent Office
Office européen des brevets

(11) Veröffentlichungsnummer: **0 000 727**
**B1**

(12) # EUROPÄISCHE PATENTSCHRIFT

(21) Anmeldenummer: **78100498.1**

(22) Anmeldetag: **26.07.78**

(51) Int. Cl.³: **C 07 D 17/10,**
A 61 K 31/425,
A 61 K 31/505,
C 07 D 275/04

(54) **3-(4-(1,3-Diazacycloalken-2-yl)-phenyl)-1,2-benzisothiazole, Verfahren zu ihrer Herstellung und diese enthaltende Arzneimittel**

(30) Priorität: **03.08.77 DE 2734882**

(43) Veröffentlichungstag der Anmeldung:
**21.02.79 Patentblatt 79/04**

(45) Bekanntmachung des Hinweises auf die Patenterteilung:
**20.08.80 Patentblatt 80/17**

(84) Benannte Vertragsstaaten:
**BE CH DE FR GB NL**

(56) Entgegenhaltungen:
**Chemical Abstracts, Vol. 58 (1963) 11340c & Ric. Sci. Rend. Ser. B 2 117—8 (1962)**

(73) Patentinhaber: **BASF Aktiengesellschaft**
**Carl-Bosch-Strasse 38**
**D - 6700 Ludwigshafen (DE)**

(72) Erfinder: **Hagen, Helmut Dr.**
**Max-Slevogt-Strasse 17e**
**D - 6710 Frankenthal (DE)**
**Lenke, Dieter, Dr.**
**Kekuleplatz 1**
**D - 6700 Ludwigshafen (DE)**
**Von Philipsborn, Gerda, Dr.**
**Naechstenbacher Weg 35**
**D - 6940 Weinheim (DE)**

EP 0 000 727 E1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 3-[4-(1,3-Diazacycloalken-2-yl)-phenyl]-1,2-benzisothiazole, Verfahren zu ihrer Herstellung und diese enthaltende Arzneimittel

Die Erfindung betrifft neue 3-[4-(1,3-Diazacycloalken-2-yl)-phenyl]-1,2-benzisothiazole und ihre Säureadditionssalze, ihre Herstellung und diese Verbindungen enthaltende pharmazeutische Zubereitungen.

Es wurde gefunden, daß Verbindungen der allgemeinen Formel I

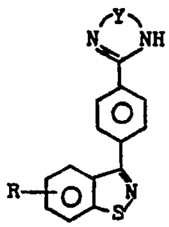

(I)

in der R ein Wasserstoffatom, einen Alkylrest mit 1 bis 4 C-Atomen, einen Alkoxyrest mit 1 bis 3 C-Atomen, ein Halogenatom oder eine Nitro-Gruppe bedeutet und Y ein Brückenglied der Formel

$$-CH_2-CH_2-,\quad -CH(CH_3)-CH_2-\ \text{oder}\ -CH_2-CH_2-CH_2-$$

darstellt, und ihre physiologisch verträglichen Säureadditionsalze wertvolle pharmakologische Eigenschaften aufweisen.

Von den genannten Bedeutungen sind als bevorzugt für R Wasserstoff, ein Halogenatom, insbesondere Chlor oder Brom, oder eine Nitrogruppe und für Y 1-Methyläthylen-1,2 und Trimethylen-1,3 zu nennen.

Als besonders bevorzugt sind für R Wasserstoff, und für Y

$$-CH(CH_3)-CH_2-\ \text{und}\ -CH_2-CH_2-CH_2-$$

hervorzuheben.

Dementsprechend sind als erfindungsgemäße Verbindungen, die summarisch als 3-[4-(1,3-Diazacyclo-2-yl)]-1,2-benzoisothiazole bezeichnet werden können, der allgemeinen Formel I beispielsweise zu nennen:

3-[4-(Imidazolin-2-yl)-phenyl]-1,2-benzisothiazol
3-[4-(Methylimidazolin-2-yl)-phenyl]-1,2-benzisothiazol
3-[4-(Tetrahydropyrimidin-2-yl)-phenyl]-1,2-benzisothiazol
5-Chlor-3-[4-(imidazolin-2-yl)-phenyl]-1,2-benzisothiazol
5-Chlor-3-[4-(methylimidazolin-2-yl)-phenyl]-1,2-benzisothiazol
4-Methoxy-3-[(4-(methylimidazolin-2-yl)-phenyl]-1,2-benzisothiazol
5-Nitro-3-[4-(imidazolin-2-yl)-phenyl]-1,2-benzisothiazol
5-Nitro-3-[4-(tetrahydropyrimidin-2-yl)-phenyl]-1,2-benzisothiazol

Die erfindungsgemäßen Verbindungen können hergestellt werden, indem man eine Verbindung der allgemeinen Formel II

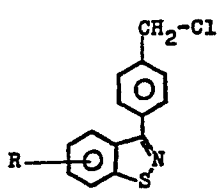

(II)

in der R die obengenannten Bedeutungen hat, mit einem Diamin der allgemeinen Formel III,

$$H_2N-Y-NH_2 \qquad \text{(III)}$$

in der Y die obengenannten Bedeutungen hat, und elementarem Schwefel zweckmäßigerweise in einem inerten organischen Lösungsmittel umsetzt und gegebenenfalls die erhaltene Verbindung in ein

physiologisch verträgliches Säureadditionssalz überführt.

Die Umsetzung wird zweckmäßig bei Temperaturen von 40 bis 150°C, bevorzugt bei Temperaturen von 70 bis 120°C, durchgeführt.

Zweckmäßige Lösungsmittel für die Umsetzung sind aromatische Kohlenwasserstoffe, insbesondere Benzolkohlenwasserstoffe, wie Benzol oder Toluol, niedere Alkohole, wie Methanol, Äthanol, Propanol, Isopropanol, Butanol oder Isobutanol, gesättigte cyclische oder aliphatische Äther, wie Dibutyläther oder Dioxan, Glykoläther, insbesondere Monoalkyläther des Glykols, wie Glykolmonomethyläther oder Glykolmonoäthyläther, oder Mischungen der genannten Lösungsmittel.

Von den genannten Lösungsmitteln sind die Benzolkohlenwasserstoffe, insbesondere Benzol und Toluol, and Monoalkyläther des Glykols, insbesondere Glykolmonomethyläther, bevorzugt.

Das Diamin der allgemeinen Formel III wird, berechnet auf die Verbindung der allgemeinen Formel II, in stöchiometrischer oder in überschüssiger Menge, gegebenenfalls bis zur 3-fachen stöchiometrischen Menge, verwendet.

Der elementare Schwefel wird, berechnet auf die verwendete Chlormethylverbindung, in stöchiometrischer oder in überschüssiger Menge bis zum 1,2-fachen verwendet. Bevorzugt wird die stöchiometrische Menge elementarer Schwefel eingesetzt.

Die Umsetzung von beispielsweise 3-(4-Chlormethylphenyl)-1,2-benzisothiazol mit Äthylendiamin und Schwefel kann durch die folgende Reaktionsgleichung beschrieben werden:

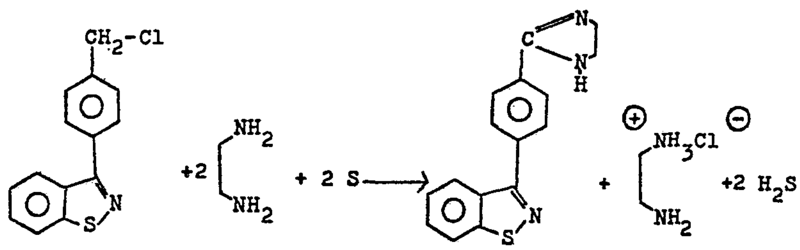

Die Ausgangsverbindungen der allgemeinen Formel II können beispielsweise durch Seitenkettenchlorierung von 3-(4-Methylphenyl)-1,2-benzisothiazolen mit Chlor bei etwa 170°C und unter UV-Bestrahlung erhalten werden.

Die 3-(4-Methylphenyl)-1,2-benzisothiazole, die den Verbindungen der allgemeinen Formel II zugrundeliegen, können erhalten werden, indem man ein o-Halogenarylketon der allgemeinen Formel IV

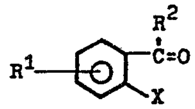

(IV)

in der $R^1$ die für R in der allgemeinen Formel I genannten Bedeutungen hat, $R^2$ einen 4-Methylphenylrest und X ein Halogenatom, insbesondere Chlor, bedeuten, mit Ammoniak und elementarem Schwefel nach dem Prinzip des Verfahrens der offengelegten deutschen Patentanmeldung P 25 03 699 umsetzt. Darin sind als Vorstufen anstelle von Verbindungen der allgemeinen Formel IV (Ketone) solche Verbindungen offenbart, in denen $R^2$ = Wasserstoff ist (Aldehyde). Die Umsetzung eines Ketons der allgemeinen Formel IV ist unten in Beispiel A.1 erläutert. Eine entsprechende Patentanmeldung wurde in der Bundesrepublik Deutschland am 3. August 1977 unter dem Aktenzeichen P 27 34 866.9 eingereicht.

Der Ausgangsstoff IV, Ammoniak und elementarer Schwefel werden in etwa stöchiometrischen Mengen verwendet, jedoch verwendet man vorzugsweise ein Verhältnis von 2 bis 10 Mol Ammoniak und von 0,9 bis 1,1 Grammatom Schwefel je Mol Ausgangsstoff IV.

Diese Umsetzung wird in der Regel bei einer Temperatur von 20 bis 250°C, vorteilhaft von 20 bis 200°C, vorzugsweise von 40 bis 180°C, insbesondere von 40 bis 120°C, drucklos oder unter Druck, kontinuierlich oder diskontinuierlich durchgeführt. Der Reaktionsdruck wird im allgemeinen durch den Gesamtdampfdruck der Komponenten bei der Umsetzungstemperatur bedingt. Gegebenenfalls kann man unter den Reaktionsbedingungen inerte organische Lösungsmittel verwenden, z.B. aromatische Kohlenwasserstoffe, wie Toluol, Äthylbenzol, o-, m-, p-Xylol, Isopropylbenzol; Alkanole und Cycloalkanole, wie Äthanol, n-Butanol, Isobutanol, Methylglykol, Cyclohexanol, Propanol, Methanol, 2-Äthylhexanol; Äther, z.B. Äthylpropyläther, Diisobutyläther, Methyl-tert.-butyläther, n-Butyläthyläther, Di-n-butyläther, Dioxan, Diisoamyläther, Diisopropyläther, Anisol, Phenetol, Cyclohexylmethyläther, Diäthyläther, Tetrahydrofuran, Thioanisol; und entsprechende Gemische. Zweckmäßig verwendet man das Lösungsmittel in einer Menge von 200 bis 10 000 Gewichtsprozent, vorzugsweise von 300 bis 1 000 Gewichtsprozent, bezogen auf Ausgangsstoff IV.

Im einzelnen kann die Reaktion wie folgt durchgeführt werden: Ausgangsstoff IV, elementarer Schwefel und Ammoniak, gegebenenfalls in Gegenwart eines Lösungsmittels, werden in einem

Druckreaktor während 3 bis 10 Stunden bei der vorgenannten Temperatur miteinander umgesetzt. Aus dem Reaktionsgemisch erhält man das 1,2-Benzisothiazol nach den üblichen Verfahren, z.B. durch fraktionierte Destillation, Filtration und gegebenenfalls anschließender Umkristallisation aus einem geeigneten Lösungsmittel, z.B. Ligroin. Man kann das Reaktionsgemisch auch nach der Entfernung überflüssigen Ammoniaks und Lösungsmittels in Wasser gießen, das gebildete Gemisch mit einem geeigneten Lösungsmittel, z.B. Methylenchlorid, Benzol, extrahieren und den Extrakt aufarbeiten.

Die erfindungsgemäßen Verbindungen der allgemeinen Formel I werden gegebenenfalls in an sich üblicher Weise in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Apfelsäure, Zitronensäure, Salicylsäure, Adipinsäure oder Benzoesäure oder können aus Fortschritte der Arzneimittelforschung Band 10, Seiten 224 bis 225, Birkhäuser Verlag, Basel und Stuttgart, 1966 entnommen werden.

Die erfindungsgemäßen Verbindungen und ihre physiologisch verträglichen Säureadditionssalze zeichnen sich durch eine starke antriarrhythmische Wirkung aus und sind besonders zur Pharmakotherapie von Herzrhythmusstörungen geeignet.

Zur Bestimmung ihrer antiarrhythmischen Wirksamkeit wurden die Substanzen Ratten (Stamm: Sprague Dawley, Gewicht: 180—240 g) 45 min vor Beginn der Narkose (Thiobutabarbital 100 mg/kg i.p.) oral appliziert.

Als arrhythmogene Substanz diente Aconitin, das 60 min nach der Substanzapplikation i.v. infundiert wurde (Dosierungsgeschwindigkeit: 0,005 mg/kg · min). Bei nichtbehandelten Tieren ($N = 30$) treten nach durchschnittlich $3,7 \pm 0,9$ min Arrhythmien auf, deren Eintritt durch Antiarrhythmica dosisabhängig verzögert werden kann.

Zur quantitativen Auswertung der linearen Beziehung zwischen log Dosis (mg/kg) der Prüfsubstanzen und der relativen Verlängerung der Aconitininfusionsdauer ($\Delta$%) wurde die Dosis bestimmt, welche die Infusionsdauer um 50% verlängert (ED 50%). Als Vergleichssubstanz diente das bekannte Antiarrhythmicum Procainamid.

Die akute Toxizität wurde an Gruppen von je 10 oder 20 weiblichen Swiss-Mäusen, Gewicht 20—26 g, bei intraperitonealer Applikation ermittelt. Als LD 50 wurde die Dosis berechnet (Probit-Analyse), nach der 50% der Tiere innerhalb von 7 Tagen starben.

Die Tabelle 1 zeigt, daß die Verbindungen der Beispiele 2 und 3, verglichen mit dem Antiarrhythmicum Procainamid, rund 5 mal stärker antiarrhythmisch wirksam sind. Ein weiterer Vorteil besteht darin, daß die Wirkung der Maximaldosis um 114 (Beispiel 2) bzw. 73% (Beispiel 3) höhere Werte erreicht, als die von Procainamid; d.h., daß der Aconitinantagonismus der geprüften Verbindungen deutlich stärker ausgeprägt ist als bei Procainamid.

Die therapeutische Breite als Quotient aus der letalen Dosis (LD 50) und der antiarrhythmisch wirkenden Dosis (ED 50%) ist 2 mal (Beispiel 2) bzw. 1,9 mal (Beispiel 3) größer als beim Procainamid.

TABELLE 1

Antiarrhythmische Wirkung und akute Toxizitat

| Verbindung | Antiarrhythmische Wirkung 1) | | | | | Akute Tox. LD 50 mg/kg | Ther. Breite 7) |
|---|---|---|---|---|---|---|---|
| | Wirksame Dosis | | Maximale Wirkung 4) | | | | |
| | ED 50% 2) | R.W. 3) | Dosis | Δ% 5) | R.M.W. 6) | | |
| Beispiel 2 | 31,2 | 5,03 | 215 | 289 | 2,14 | 126 | 4,03 |
| Beispiel 3 | 32,7 | 4,80 | 215 | 233 | 1,73 | 90,5 | 2,76 |
| Procainamid | 157 | 1,00 | 681 | 135 | 1,00 | 227 | 1,45 |

1) Aconitinarrhythmie Ratte.
2) Dosis (mg/kg) per os, welche die Aconitininfusionsdauer (min) um 50 [%] verlängert
3) R.W. = Relative Wirksamkeit; Procainamid = 1,00
4) Wirkung der höchsten nicht toxischen Dosis
5) Verlängerung der Aconitininfusionsdauer Δ%
6) R.M.W. = Relative maximale Wirksamkeit
7) $\frac{LD\ 50}{ED\ 50\ \%}$

In Testmodellen auf antiarrhythmische Wirkung wurden folgende Befunde erhoben:

Reizzeitspannungsbeziehung (isol. Vorhof):
Beispiel 2 (Tetrahydropyrimidinderivat): 1,24-fache Chinidinwirkung
Beispiel 3 (Methylimidazolinderivat): 1,5-fache Chinidinwirkung

Flimmerschwelle am Meerschweinchen:
Beispiel 2: i.v. = 1,8-fache Chinidinwirkung

Maximale Folgefrequenz am unnarkotisierten Kaninchen:
Beispiel 2: i.v. = 7-fache Chinidinwirkung.

Gegenstand der Erfindung sind demnach auch therapeutische Mittel oder Zubereitungen, die neben üblichen Träger- und Verdünnungsmittel eine Verbindung der allgemeinen Formel I oder dessen physiologisch verträgliches Säureadditionssalz als Wirkstoff enthalten sowie die Verwendung der neuen Verbindungen zu therapeutischen Zwecken.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungsformen, wie Injektionslösungen oder Zusätze zu Infusionslösungen, in Betracht. Weiterhin seien als Zubereitungen beispielsweise auch Suppositorien genannt.

Die entsprechenden Tabletten können beispielsweise durch Mischen des Wirkstoffs mit bekannten Hilfsstoffen, beispielsweise inerte Verdünnungsmittel, wie Dextrose, Zucker, Sorbit, Mannit, Polyvinylpyrrolidon, Kalziumcarbonat, Kalziumphosphat oder Milchzucker, Sprengmitteln, wie Mais, Stärke, Alginsäure oder Polyvinylpyrrolidon, Bindemitteln, wie Stärke oder Gelatine, Gleitmitteln, wie Magnesiumstearat oder Talkum und/oder Mitteln zur Erzielung des Depoteffektes, wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmackverbessernde Mittel, wie Saccharin, Cyclamat oder Zucker sowie beispielsweise Aromastoffe, wie Vanillin oder Orangenextrakt, enthalten. Sie können außerdem Suspendierhilfsstoffe, wie Natriumcarboxymethylcellulose oder Konservierungsstoffe, wie Parahydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenem Trägermaterial, wie Neutralfetten oder Polyäthylenglykolen bzw. dessen Derivaten, herstellen.

Die Einzeldosis einer erfindungsgemäßen Substanz am Menschen liegt bei 5 bis 100 mg, vorzugsweise bei 10 bis 80 mg.

Die Erfindung wird durch die nachfolgenden Ausführungsbeispiele näher erläutert.

A. Beispiele für die Herstellung von Ausgangsverbindungen

Beispiel 1

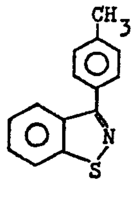

In einem Emailleautoklav werden 230,5 Teile 2-Chlor-4'-methyl-benzophenon, 32 Teile Schwefel und 100 Teile $NH_3$ in 800 Teilen Methylglykol 6 Stunden bei 160°C umgesetzt. Man erhält 200 Teile 3-(4'-Methylphenyl)-1,2-benzisothiazol mit Fp 56°C. Die Ausbeute entspricht 89% der Theorie.

In entsprechender Weise wird aus 2,5-Dichlor-4'-methylbenzophenon, im selben Molverhältnis

und unter denselben Bedingungen mit Schwefel und Ammoniak umgesetzt, 5-Chlor-3-(4'-methylphenyl)-1,2-benzisothiazol vom Fp. 121°C in 85% Ausbeute erhalten.

Aus 2-Chlor-5-nitro-4-methylbenzophenon wird 5-Nitro-3-(4'-methylphenyl)-1,2-benzisothiazol vom Fp. 179°C in 90% Ausbeute erhalten.

Beispiel 2

3-(4'-Chlormethylphenyl)-1,2-benzisothiazol

225 g 3-(4'-Methylphenyl)-1,2-benzisothiazol werden in einer Rührapparatur auf 170°C erhitzt und unter Bestrahlung mit einer UV-Lampe werden innerhalb von 2 Stunden 100 g Chlor eingeleitet. Der Endpunkt der Reaktion wird gaschromatographisch bestimmt (Verschwinden des Ausgangsprodukts). Anschließend wird das Reaktionsgemisch abgekühlt, abgesaugt und aus Methanol umkristallisiert. Man erhält 208 g 3-(4-Chlormethylphenyl)-1,2-benzisothiazol von einem Schmelzpunkt von 86 bis 89°C, was einer Ausbeute von 80% der Theorie entspricht.

In entsprechender Weise wird 5-Chlor-3-(4'-Chlormethylphenyl)-1,2-benzisothiazol vom Fp. 116°C mit 78% Ausbeute hergestellt.

B. Erfindungsgemäße Verbindungen

Beispiel 1

3-[4-Imidazolin-2-yl-phenyl]-1,2-benzisothiazol

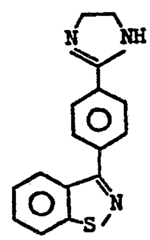

26 g 3-(4-Chlormethylphenyl)-1,2-benzisothiazol, 6,4 g Schwefel und 300 ml Toluol werden auf 50°C erwärmt und bei dieser Temperatur 12 g Äthylendiamin langsam zugegeben. Anschließend wird das Reaktionsgemisch 15 Stunden bei Rückflußtemperatur gerührt, heiß abfiltriert und das Filtrat auf 10° bis 15°C abgekühlt. Man erhält 21 g 3-(4-Imidazolin-2-yl-phenyl)-1,2-benzisothiazol mit einem Schmelzpunkt von 177°C. Das entspricht einer Ausbeute von 75% der Theorie.

| | | C | H | N | S |
|---|---|---|---|---|---|
| Analyse: | ber. | 68,8 | 4,6 | 15,1 | 11,5 |
| | gef. | 68,4 | 4,7 | 15,0 | 11,7 |

Das Hydrochlorid der Verbindung hat einen Schmelzpunkt von 318°C.

Beispiel 2

3-[4-(Tetrahydropyrimidin-2-yl)-phenyl]-1,2-benzisothiazolhydrochlorid

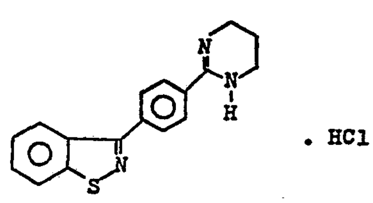

26 g 3-(4-Chlormethylphenyl)-1,2-benzisothiazol, 6,4 g Schwefel und 300 ml Toluol werden auf 50°C erwärmt. Bei dieser Temperatur werden 15 g 1,3-Diaminopropan langsam zugegeben. Das Reaktionsgemisch wird noch 20 Stunden bei Rückflußtemperatur gerührt. Anschließend werden innerhalb 1 Stunde 20 g Chlorwasserstoffgas eingeleitet, es wird auf Raumtemperaturen abgekühlt und der entstandene Feststoff abgesaugt. Nach dem Umkristallisieren aus Wasser unter Zusatz von Aktivkohle erhält man 18 g 3-[4-(Tetrahydropyrimidin-2-yl)-phenyl]-1,2-benzisothiazol-hydrochlorid mit einem Schmelzpunkt von 314°C (Z). Die Ausbeute entspricht 54,6% der Theorie.

| | | C | H | N | S | Cl |
|---|---|---|---|---|---|---|
| Analyse: | ber. | 61,9 | 4,9 | 12,7 | 9,7 | 10,8 |
| | gef. | 61,4 | 5,1 | 12,4 | 9,9 | 10,9 |

Beispiel 3

3-[4-(4-Methylimidazolin-2-yl)-phenyl]-1,2-benzisothiazol-hydrochlorid

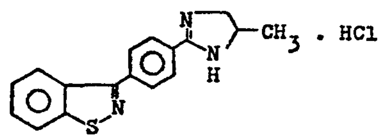

26 g 3-(4'-Chlormethylphenyl)-1,2-benzisothiazol, 6,4 g Schwefel und 15 g 1,2-Diaminopropan werden in 400 ml Glykolmonomethyläther 15 Stunden auf 110°C erwärmt. Nach dem Abdestillieren des Lösungsmittels wird der Rückstand in 50 ml Methanol gelöst und unter Kühlen in 400 ml ätherische Salzsäure (15 g HCl in Diäthyläther) eingerührt. Die gebildeten Kristalle werden abgesaugt, in 300 ml Wasser gelöst, filtriert, das Filtrat mit conc. NaOH alkalisch gestellt und mit Methylenchlorid extrahiert. Die Methylenchloridphase wird getrocknet und 20 g Chlorwasserstoffgas eingeleitet. Man erhält 17 g des gewünschten Endstoffs als farblose Kristalle vom Schmelzpunkt 243°C. Die Ausbeute entspricht 52% der Theorie.

| | | C | H | N | S | Cl |
|---|---|---|---|---|---|---|
| Analyse: | ber. | 61,9 | 4,9 | 12,7 | 9,7 | 10,8 |
| | gef. | 61,8 | 5,0 | 12,6 | 9,7 | 10,8 |

Beispiel 4

5-Chlor-3-[4-(imidazolin-2-yl)-phenyl]-1,2-benzisothiazol

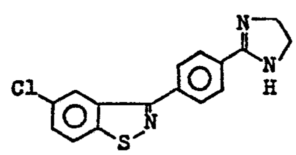

44 g 5-Chlor-3-(4'-chlormethylphenyl)-1,2-benzisothiazol, 9,6 g Schwefel und 18,0 g Äthylendiamin werden in 500 ml Toluol 20 Stunden unter Rückfluß gekocht. Das Reaktionsgemisch wird heiß filtriert, das Filtrat abgekühlt und die gebildeten Kristalle werden abgesaugt. Nach dem Umkristallisieren aus Toluol unter Zusatz von Aktivkohle werden 26 g 5-Chlor-3-[4-(imidazolin-2-yl)-phenyl]-1,2-benzisothiazol vom Schmelzpunkt 195°C erhalten. Die Ausbeute entspricht 55% der Theorie.

| | | C | H | N | S | Cl |
|---|---|---|---|---|---|---|
| Analyse: | ber. | 61,2 | 3,8 | 13,4 | 10,2 | 11,3 |
| | gef. | 61,0 | 3,9 | 13,3 | 10,3 | 11,4 |

Beispiel 5

5-Chlor-3-[4-(4-methylimidazolin-2-yl)-phenyl]-1,2-benzisothiazol-hydrochlorid

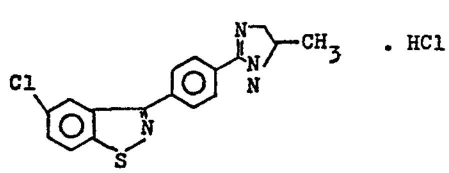

44 g 5-Chlor-3-(4'-chlormethylphenyl)-1,2-benzisothiazol, 9,6 g Schwefel und 22,5 g 1,2-Diaminopropan werden in 600 ml Toluol 24 Stunden unter Rückfluß gekocht. Das Reaktionsgemisch wird heiß filtriert und am Rotationsverdampfer eingeengt. Der Rückstand wird in 500 ml Äther gelöst

und 30 g Chlorwasserstoffgas unter Kühlen eingeleitet. Die gebildeten Kristalle werden abgesaugt und aus Wasser umkristallisiert. Man erhält 31 g, Schmelzpunkt 248°C (Z). Die Ausbeute entspricht 57% der Theorie.

| | | C | H | N | S | Cl |
|---|---|---|---|---|---|---|
| Analyse: | ber. | 56,0 | 4,1 | 11,5 | 8,8 | 19,5 |
| | gef. | 55,8 | 4,2 | 11,4 | 8,7 | 19,6 |

Formulierungsbeispiele, die in üblicher Weise hergestellt werden

1. *Tabletten:*

| | | |
|---|---|---|
| a) | Ein Wirkstoff der allgemeinen Formel I | 5 mg |
| | Lactose | 200 mg |
| | Methylcellulose | 15 mg |
| | Maisstärke | 50 mg |
| | Talkum | 11 mg |
| | Magnesiumstearat | 4 mg |
| | | 285 mg |
| b) | Ein Wirkstoff der allgemeinen Formel I | 20 mg |
| | Lactose | 178 mg |
| | Avicel | 80 mg |
| | Polywachs 6000 | 20 mg |
| | Magnesiumstearat | 2 mg |
| | | 300 mg |
| c) | Ein Wirkstoff der allgemeinen Formel I | 50 mg |
| | Polyvinylpyrrolidon (mitt. M.G. 25 000) | 170 mg |
| | Polyäthylenglykol (mittl. M.G. 4000) | 14 mg |
| | Hydroxypropylmethylcellulose | 40 mg |
| | Talkum | 4 mg |
| | Magnesiumstearat | 2 mg |
| | | 280 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4000), Hydroxypropylmethylcellulose, Talkum und Magnesiumstearat vermischt und zu Tabletten à 280 mg verpreßt.

2. *Beispiel für Dragees:*

| | |
|---|---|
| Ein Wirkstoff der allgemeinen Formel I | 60 mg |
| Lactose | 90 mg |
| Maisstärke | 60 mg |
| Polyvinylpyrrolidon | 6 mg |
| Magnesiumstearat | 1 mg |
| | 217 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. *Kapselformulierung:*

| | |
|---|---|
| Ein Wirkstoff der allgemeinen Formel I | 5,0 mg |
| Magnesiumstearat | 2,0 mg |
| Milchzucker | 19,3 mg |

4. *Injektionslösung:*

| | |
|---|---|
| Ein Wirkstoff der allgemeinen Formel I | 10 mg |
| Natriumchlorid | 9 mg |
| destilliertes Wasser, q.s. auf | 1,0 ml |

**Patentansprüche**

1. Verbindungen der allgemeinen Formel I

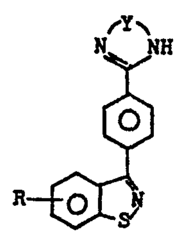

(I)

in der R ein Wasserstoffatom, einen Alkylrest mit 1 bis 4 C-Atomen, einen Alkoxyrest mit 1 bis 3 C-Atomen, ein Halogenatom oder eine Nitro-Gruppe bedeutet und Y ein Brückenglied der Formel

$$-CH_2-CH_2-,\ -CH(CH_3)-CH_2-\ \text{oder}\ -CH_2-CH_2-CH_2-$$

darstellt und ihre physiologisch verträglichen Säureadditionssalze.

2. 3-[4-(Tetrahydropyrimidin-2-yl)-phenyl]-1,2-benzisothiazol und dessen physiologisch verträglichen Säureadditionssalze.

3. 3-[4-(4-Methylimidazolin-2-yl)-phenyl]-1,2-benzisothiazol und dessen physiologisch verträglichen Säureadditionssalze.

4. Verfahren zur Herstellung von Verbindungen der allgemeinen Formel I, dadurch gekennzeichnet, daß man eine Verbindung der allgemeinen Formel II

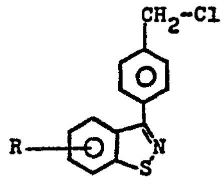

(II)

in der R die im Anspruch 1 angegebenen Bedeutungen hat, mit einem Diamin der allgemeinen Formel III

$$H_2N—Y—NH_2 \qquad (III)$$

in der Y die in Anspruch 1 angegebenen Bedeutungen hat, und elementarem Schwefel zweckmäßigerweise in einem inerten organischen Lösungsmittel umsetzt.

5. Pharmazeutische Zubereitungen, dadurch gekennzeichnet, daß sie neben üblichen pharmazeutischen Träger- und Verdünnungsmitteln eine Verbindung der allgemeinen Formel I als Wirkstoff enthalten.

6. Pharmazeutische Zubereitungen mit antiarrhythmischer Wirkung, enthaltend neben üblichen pharmazeutischen Träger- und Verdünnungsmitteln die Verbindung 3-[4-(Tetrahydropyrimidin-2-yl)-phenyl]-1,2-benzisothiazol oder dessen physiologisch verträgliches Säureadditionsalz.

7. Pharmazeutische Zubereitungen mit antiarrhythmischer Wirkung, enthaltend neben üblichen pharmazeutischen Träger- und Verdünnungsmitteln die Verbindung 3-[4-(Methylimidazolin-2-yl)-phenyl]-1,2-benzisothiazol oder dessen physiologisch verträgliches Säureadditionssalz.

**Revendications**

1. Composés de formule générale I

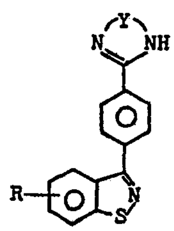

(I)

dans laquelle R est un atome d'hydrogène, un radical alkyle à 1 à 4 atomes de carbone, un radical alcoxy à 1 à 3 atomes de carbone, un atome d'halogène ou un groupe nitro et Y est un pont de formule

$$—CH_2—CH_2—,\ —\underset{\displaystyle CH_3}{\underset{|}{CH}}—CH_2—\ \text{ou}\ —CH_2—CH_2—CH_2—$$

et leurs sels d'addition acides tolérés physiologiquement.

2. [(Tétrahydropyrimidine-2-yl)-4-phényl]-3-benzisothiazole-1,2 et ses sels d'addition acides physiologiquement acceptables.

3. [(Méthyl-4-imidazoline-2-yl)-4-phényl]-3-benzisothiazole-1,2 et ses sels d'addition acides physiologiquement acceptables.

4. Procédé de préparation de composés de formule générale I, caractérisé par le fait qu'on fait réagir un composé de formule générale II

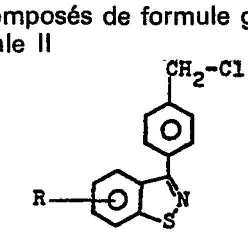

(II)

dans laquelle R a le sens indiqué dans la revendication 1, avec une diamine de formule générale III

$$H_2N—Y—NH_2 \qquad (III)$$

dans laquelle Y a le sens indiqué dans la revendication 1, et du soufre élémentaire, de préférence dans un solvant organique inerte.

5. Préparations pharmaceutiques caractérisés par le fait qu'elles contiennent, outre des supports et diluants pharmaceutiques usuels, un composé de formule générale I comme matière active.

6. Préparations pharmaceutiques à effet anti-arythmique contenant, outre des supports et diluants pharmaceutiques usuels, le composé [(Tétrahydropyrimidine-2-yl)-4-phényl]-3-benzisothiazole-1,2-ou un sel d'addition acide physiologiquement acceptable de ce composé.

7. Préparations pharmaceutiques à effet anti-arythmique contenant, outre des supports et diluants pharmaceutiques usuels, le composé [(Méthylimidazoline-2-yl)-4-phényl]-3-benzisothiazole-1,2 ou un sel d'addition acide physiologiquement acceptable de ce composé.

## Claims

1. A compound of the general formula I

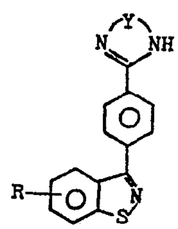

(I)

where R is hydrogen, alkyl of 1 to 4 carbon atoms, alkoxy of 1 to 3 carbon atoms, halogen or nitro and Y is a bridge member of the formula

$$-CH_2-CH_2-,\quad -\underset{\underset{\displaystyle CH_3}{|}}{C}H-CH_2-\quad \text{or}\quad -CH_2-CH_2-CH_2-$$

and their physiologically acceptable addition salts with acids.

2. 3-[Tetrahydropyrimidin-2-yl)-phenyl]-1,2-benzisothiazole and its physiologically acceptable addition salts with acids.

3. 3-[4-(4-Methylimidazolin-2-yl)-phenyl]-1,2-benzisothiazole and its physiologically acceptable addition salts with acids.

4. A process for the manufacture of a compound of the general formula I, characterized in that a compound of the general formula II

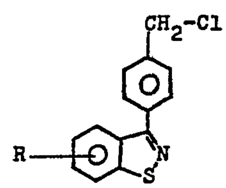

(II)

where R has the meanings given in claim 1, is reacted with a diamine of the general formula III

$$H_2N-Y-NH_2 \qquad (III)$$

where Y has the meanings given in claim 1, and with elementary sulfur, advantageously in an inert organic solvent.

5. A pharmaceutical formulation characterized in that it contains a compound of the general formula I as the active ingredient, together with conventional pharmaceutical excipients and diluents.

6. A pharmaceutical formulation having an antiarrhythmic effect, which contains the compounds 3-[4-(tetra-hydropyrimidin-2-yl)-phenyl]-1,2-benzisothiazole or one of its physiologically acceptable addition salts with an acid, in addition to conventional pharmaceutical excipients and diluents.

7. A pharmaceutical formulation having an antiarrhythmic effect, which contains the compound 3-[4-(4-methylimidazolin-2-yl)-phenyl]-1,2-benzisothiazole or one of its physiologically acceptable addition salts with an acid, in addition to conventional pharmaceutical excipients and diluents.